# EUROPEAN PATENT APPLICATION

(11) **EP 3 888 643 A1**
(43) Date of publication of application: **06.10.2021**
(21) Application number: 19888521.2
(22) Date of filing: 25.11.2019
(51) Int. Cl.: A61K 31/138, A61K 31/519, A61K 31/565, A61K 45/00, A61P 35/00, A61P 43/00

(54) **THERAPEUTIC AND PROPHYLACTIC METHOD FOR TUMOR TREATABLE WITH ENDOCRINE THERAPY BY COMBINED USE OF FIBROBLAST GROWTH FACTOR RECEPTOR INHIBITOR WITH ENDOCRINE THERAPY**

(30) Priority: 26.11.2018 JP 2018220046
(71) Applicant: Taiho Pharmaceutical Co., Ltd., Chiyoda-ku Tokyo 101-8444 (JP)
(72) Inventor: HIRAI, Hiroshi, Tokyo 101-0047 (JP); MIURA, Akihiro, Tsukuba-shi, Ibaraki 300-2611 (JP); SOOTOME, Hiroshi, Tsukuba-shi, Ibaraki 300-2611 (JP)
(74) Representative: Hoffmann Eitle
(86) International application number: PCT/JP2019/045901
(87) International publication number: WO 2020/110974

(57) **Abstract**

Provided is a novel treatment method for cancer tumors to which endocrine therapy is to be applied, using an FGFR inhibitor (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof. Further provided are a pharmaceutical composition comprising the above compound or a pharmaceutically acceptable salt thereof for use in treatment and/or prevention of tumors to which endocrine therapy is to be applied, the pharmaceutical composition being used in combination with endocrine therapy, as well as its related compound, use, method, and combination.

## Description

### Technical Field

### Cross-Reference to Related Application

This application claims priority based on Japanese Patent Application No. 2018-220046 filed on November 26, 2018, the entire contents of which are incorporated herein by reference. The present disclosure relates to treatment and prevention of tumors to which endocrine therapy is to be applied.

### Background Art

Fibroblast growth factors (FGFs) are expressed in various tissues, and are one of the growth factors that regulate cell proliferation and differentiation. The physiological activity of the FGFs is mediated by fibroblast growth factor receptors (FGFRs), which are specific cell surface receptors. FGFRs belong to a receptor protein tyrosine kinase family, and comprise an extracellular ligand-binding domain, a single transmembrane domain, and an intracellular tyrosine kinase domain. Four types of FGFRs (FGFR1, FGFR2, FGFR3, and FGFR4) have been heretofore identified. FGFRs bind to FGFs to form dimers, and are activated by phosphorylation. Activation of the receptors induces mobilization and activation of specific downstream signal transduction molecules, thereby developing physiological functions. Some reports have been made about the relationship between aberrant FGF/FGFR signaling and various human tumors. Aberrant activation of FGF/FGFR signaling in human tumors is considered to be attributable to overexpression of FGFRs and/or gene amplification, gene mutation, chromosomal translocation, insertion, and inversion, gene fusion, or an autocrine or paracrine mechanism by overproduction of FGFs (ligands) (NPL 1, 2, 3, and 4).

In some types of tumors in hormone-responsive tissues, such as the mammary gland and prostate gland, hormonal stimulation of these tissues promotes tumor growth. Focusing on this point, therapy by controlling hormonal stimulation of tumors is known as endocrine therapy (also called "hormone therapy"). For example, for estrogen receptor-positive breast tumors, the application of endocrine therapies, such as suppression of estrogen-producing ovarian function (by surgical procedures, radiation procedures, or drug administration), and estrogen-producing enzyme (aromatase) inhibitors and estrogen receptor inhibitors (antiestrogens), is considered. Further, among prostate tumors, for androgen-dependent prostatic tumors, endocrine therapies, such as suppression of testosterone secretion (resection of the testes, which are reproductive organs, or drug administration of LH-RH agonists, LH-RH antagonists, estrogenic drugs, or the like) and administration of antiandrogens, are known. Antiandrogens are also used for benign prostate hyperplasia (BPH). In addition, LH-RH agonists are also used for ovarian cancer in some cases.

Aberrant FGF/FGFR signaling is also found in tumors in these hormone-responsive tissues and is reportedly associated with tumors. In ER-positive breast cancer patients, a correlation between FGFR1 amplification and poor prognosis has been reported (NPL 5). In prostate cancer, aberrant expression of the FGF/FGFR system leads to constitutive activation of multiple downstream signaling pathways and is involved in cancer progression (NPL 6). Overexpression of FGFR1 localized in stromal cells has been reported in benign prostate hyperplasia (NPL 7). Further, for example, FGFR1 gene amplification has been reported in ovarian cancer (NPL 1).

In addition, regarding endocrine therapy for hormone-responsive tissue cancer, there is a known phenomenon called "resistance to endocrine therapy," in which patients initially show a suppressive response to progression, but later lose such a response. The FGF/FGFR system is reportedly involved in such resistance to endocrine therapy. For example, FGFR1 gene amplification in breast cancer results in FGFR1 overexpression and activates FGFR1 downstream signaling in a ligand-dependent and -independent manner (NPL 4), and facilitates gene expression downstream of the estrogen receptor in the absence of estrogen stimulation, resulting in resistance to endocrine therapy (NPL 8). Moreover, in prostate cancer, resistance to androgen deprivation therapy is sometimes observed with neuroendocrine tumor-like differentiation without expression of the androgen receptor. In addition, there is a double-negative phenotype that neither expresses the androgen receptor nor exhibits neuroendocrine tumor characteristics. It has been reported that constitutive activation of FGFs and downstream signaling pathways avoids androgen receptor dependence (NPL 9). In the emergence of resistance to endocrine therapy, activation of MAPK etc. downstream of the FGFR signaling pathway has been commonly found (NPL 4 and NPL 9).

In cancer to which endocrine therapy is to be applied, several FGFR inhibitors are in clinical development for use in combination with hormonal drugs. Regarding breast cancer, a phase II study of dovitinib (an inhibitor of multi-kinases including FGFR kinase) in combination with fulvestrant (an antiestrogen) showed that in a subpopulation of FGF pathway-amplified patients, the median value of the mean progression-free survival (PFS) was significantly improved in the combination group, compared with the group administered with fulvestrant alone (10.9 vs 5.5 months) (NPL 10). For breast cancer, a phase II study of AZD4547 (a pan-FGFR inhibitor) is underway in a combined group with fulvestrant or exemestane (an aromatase inhibitor) and in a fulvestrant and placebo administration group (NCT01202591) (NPL 11). For prostate cancer, a phase II study of dovitinib in combination with androgen deprivation therapy (NCT02065323) and a phase II study of dovitinib in combination with abiraterone (an androgen synthesis enzyme inhibitor) (NCT01994590) are underway.

PTL 1 reported about disubstituted benzene alkynyl compounds having an FGFR inhibitory effect. PTL 2 and 3 respectively reported that these compounds are effective against cancer with a specific FGFR2 mutation, and that an intermittent administration schedule may be effective. PTL 4 reported that cancer treatment in combination with antitumor agents can be performed.

### Citation List

### Patent Literature

PTL 1: WO2013/108809
PTL 2: WO2015/008844
PTL 3: WO2015/008839
PTL 4: WO2017/150725

### Non-patent Literature

NPL 1: Nat. Rev. Cancer 10: 116-129 (2010)
NPL 2: J. Clin. Oncol. 24, 3664-3671 (2006)
NPL 3: Mol. Cancer Res. 3, 655-667 (2005)
NPL 4: Cancer Res. 70, 2085-2094 (2010)
NPL 5: Breast Cancer Res 2007; 9: R23
NPL 6: Clin Cancer Res 19: 5856-5866 (2013)
NPL 7: Eur J Endocrinol 145: 303-310 (2001)
NPL 8: Clin Cancer Res 23: 6138-6150 (2017)
NPL 9: Cancer Cell 32: 474-489 (2017)
NPL 10: Breast Cancer Research. 19, 18 (2017)
NPL 11: The Breast. 37, 126-133 (2018)
NPL 12: Oncotarget. 8: 16052-16074 (2017)

### Summary of Invention

### Summary of Disclosure

### Technical Problem

An object of the present disclosure is to provide a novel treatment method for tumors to which endocrine therapy is to be applied, the method showing a significantly excellent antitumor effect and using an FGFR inhibitor.

### Solution to Problem

In the present disclosure, when the combined use of administration of an FGFR inhibitor (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof and endocrine therapy was examined for tumors to which endocrine therapy was to be applied, it was found that the antitumor effect was significantly enhanced compared with the single use of the administration or endocrine therapy.

That is, embodiments of the present disclosure provide the following [A1] to [A16], [B1] to [B14], [C1] to [C14], [D1] to [D14], and [E1] to [E14].
[A1] A pharmaceutical composition for use in treatment and/or prevention of a tumor to which endocrine therapy is to be applied, the pharmaceutical composition comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof, and the pharmaceutical composition being administered in combination with endocrine therapy.
[A2] The pharmaceutical composition according to [A1], wherein the tumor is a tumor expressing FGFR.
[A3] The pharmaceutical composition according to [A1] or [A2], wherein the tumor is a tumor with activated FGFR signaling.
[A4] The pharmaceutical composition according to any one of [A1] to [A3], wherein the tumor is a tumor having an FGFR genetic aberration.
[A5] The pharmaceutical composition according to any one of [A1] to [A4], wherein the tumor to which endocrine therapy is to be applied is selected from the group consisting of breast cancer, prostate cancer, ovarian cancer, and benign prostate hyperplasia.
[A6] The pharmaceutical composition according to any one of [A1] to [A5], wherein the tumor is a tumor resistant to endocrine therapy.
[A7] The pharmaceutical composition according to any one of [A1] to [A6], wherein the tumor is breast cancer expressing FGFR1.
[A8] The pharmaceutical composition according to any one of [A1] to [A7], wherein the tumor is breast cancer having FGFR1 gene amplification.
[A9] The pharmaceutical composition according to any one of [A1] to [A8], wherein the tumor to which endocrine therapy is to be applied is breast cancer, and the endocrine therapy comprises at least one member selected from the group consisting of ovarian function suppression, aromatase inhibitor administration, antiestrogen administration, and progestogen administration.
[A10] The pharmaceutical composition according to [A9], wherein the endocrine therapy is antiestrogen administration.
[A11] The pharmaceutical composition according to [A10], wherein the antiestrogen is tamoxifen, fulvestrant, or a salt or derivative thereof.
[A12] The pharmaceutical composition according to any one of [A1] to [A11], wherein endocrine therapy and administration of the composition comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof are performed simultaneously, separately, or continuously.
[A13] The pharmaceutical composition according to any one of [A1] to [A12], which is administered to a tumor patient who has undergone endocrine therapy.
[A14] The pharmaceutical composition according to any one of [A1] to [A13], which is administered to a tumor patient who will undergo endocrine therapy after administration of the pharmaceutical composition.
[A15] A method for treating and/or preventing a tumor to which endocrine therapy is to be applied, comprising administering an effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof to a patient, in combination with endocrine therapy.
[A16] A combination of endocrine therapy and administration of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof for use in treatment and/or prevention of a tumor to which endocrine therapy is to be applied.
[B1] (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof for use in treatment and/or prevention of a tumor to which endocrine therapy is to be applied, being administered in combination with endocrine therapy.
[B2] The compound or a pharmaceutically acceptable salt thereof according to [B1], wherein the tumor is a tumor expressing FGFR.
[B3] The compound or a pharmaceutically acceptable salt thereof according to [B1] or [B2], wherein the tumor is a tumor with activated FGFR signaling.
[B4] The compound or a pharmaceutically acceptable salt thereof according to any one of [B1] to [B3], wherein the tumor is a tumor having an FGFR genetic aberration.
[B5] The compound or a pharmaceutically acceptable salt thereof according to any one of [B1] to [B4], wherein the tumor to which endocrine therapy is to be applied is selected from the group consisting of breast cancer, prostate cancer, ovarian cancer, and benign prostate hyperplasia.
[B6] The compound or a pharmaceutically acceptable salt thereof according to any one of [B1] to [B5], wherein the tumor is a tumor resistant to endocrine therapy.
[B7] The compound or a pharmaceutically acceptable salt thereof according to any one of [B1] to [B6], wherein the tumor is breast cancer expressing FGFR1.
[B8] The compound or a pharmaceutically acceptable salt thereof according to any one of [B1] to [B7], wherein the tumor is breast cancer having FGFR1 gene amplification.
[B9] The compound or a pharmaceutically acceptable salt thereof according to any one of [B1] to [B8], wherein the tumor to which endocrine therapy is to be applied is breast cancer, and the endocrine therapy comprises at least one member selected from the group consisting of ovarian function suppression, aromatase inhibitor administration, antiestrogen administration, and progestogen administration.
[B10] The compound or a pharmaceutically acceptable salt thereof according to [B9], wherein the endocrine therapy is antiestrogen administration.
[B11] The compound or a pharmaceutically acceptable salt thereof according to [B10], wherein the antiestrogen is tamoxifen, fulvestrant, or a salt or derivative thereof.
[B12] The compound or a pharmaceutically acceptable salt thereof according to any one of [B1] to [B11], wherein endocrine therapy and administration of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof are performed simultaneously, separately, or continuously.
[B13] The compound or a pharmaceutically acceptable salt thereof according to any one of [B1] to [B12], which is administered to a tumor patient who has undergone endocrine therapy.
[B14] The compound or a pharmaceutically acceptable salt thereof according to any one of [B1] to [B13], which is administered to a tumor patient who will undergo endocrine therapy after administration of the compound or a pharmaceutically acceptable salt thereof.
[C1] Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof for the manufacture of a medicament for use in treatment and/or prevention of a tumor to which endocrine therapy is to be applied, being administered in combination with endocrine therapy.
[C2] The use according to [C1], wherein the tumor is a tumor expressing FGFR.
[C3] The use according to [C1] or [C2], wherein the tumor is a tumor with activated FGFR signaling.
[C4] The use according to [C1] or [C2], wherein the tumor is a tumor having an FGFR genetic aberration.
[C5] The use according to [C1] or [C2], wherein the tumor to which endocrine therapy is to be applied is selected from the group consisting of breast cancer, prostate cancer, ovarian cancer, and benign prostate hyperplasia.
[C6] The use according to [C1] or [C2], wherein the tumor is a tumor resistant to endocrine therapy.
[C7] The use according to [C1] or [C2], wherein the tumor is breast cancer expressing FGFR1.
[C8] The use according to [C1] or [C2], wherein the tumor is breast cancer having FGFR1 gene amplification.
[C9] The use according to [C1] or [C2], wherein the tumor to which endocrine therapy is to be applied is breast cancer, and the endocrine therapy comprises at least one member selected from the group consisting of ovarian function suppression, aromatase inhibitor administration, antiestrogen administration, and progestogen administration.
[C10] The use according to [C9], wherein the endocrine therapy is antiestrogen administration.
[C11] The use according to [C10], wherein the antiestrogen is tamoxifen, fulvestrant, or a salt or derivative thereof.
[C12] The use according to any one of [C1] to [C11], wherein endocrine therapy and administration of a medicament comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof are performed simultaneously, separately, or continuously.
[C13] The use according to any one of [C1] to [C12], wherein administration is performed on a tumor patient who has undergone endocrine therapy.
[C14] The use according to any one of [C1] to [C13], wherein the medicament is administered to a tumor patient who will undergo endocrine therapy after administration of the medicament.
[D1] A method for treating and/or preventing a tumor to which endocrine therapy is to be applied, comprising administering an effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof to a patient, in combination with endocrine therapy.
[D2] The method according to [D1], wherein the tumor is a tumor expressing FGFR.
[D3] The method according to [D1] or [D2], wherein the tumor is a tumor with activated FGFR signaling.
[D4] The method according to any one of [D1] to [D3], wherein the tumor is a tumor having an FGFR genetic aberration.
[D5] The method according to any one of [D1] to [D4], wherein the tumor to which endocrine therapy is to be applied is selected from the group consisting of breast cancer, prostate cancer, ovarian cancer, and benign prostate hyperplasia.
[D6] The method according to any one of [D1] to [D5], wherein the tumor is a tumor resistant to endocrine therapy.
[D7] The method according to any one of [D1] to [D6], wherein the tumor is breast cancer expressing FGFR1.
[D8] The method according to any one of [D1] to [D7], wherein the tumor is breast cancer having FGFR1 gene amplification.
[D9] The method according to any one of [D1] to [D8], wherein the tumor to which endocrine therapy is to be applied is breast cancer, and the endocrine therapy comprises at least one member selected from the group consisting of ovarian function suppression, aromatase inhibitor administration, antiestrogen administration, and progestogen administration.
[D10] The method according to [D9], wherein the endocrine therapy is antiestrogen administration.
[D11] The method according to [D10], wherein the antiestrogen is tamoxifen, fulvestrant, or a salt or derivative thereof.
[D12] The method according to any one of [D1] to [D11], wherein endocrine therapy and administration of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof are performed simultaneously, separately, or continuously.
[D13] The method according to any one of [D1] to [D12], wherein administration is performed on a tumor patient who has undergone endocrine therapy.
[D14] The method according to any one of [D1] to [D13], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof is administered to a tumor patient who will undergo endocrine therapy after administration of the compound or a pharmaceutically acceptable salt thereof.
[E1] A combination of endocrine therapy and administration of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof, for use in treatment and/or prevention of a tumor to which endocrine therapy is to be applied.
[E2] The combination according to [E1], wherein the tumor is a tumor expressing FGFR.
[E3] The combination according to [E1] or [E2], wherein the tumor is a tumor with activated FGFR signaling.
[E4] The combination according to any one of [E1] to [E3], wherein the tumor is a tumor having an FGFR genetic aberration.
[E5] The combination according to any one of [E1] to [E4], wherein the tumor to which endocrine therapy is to be applied is selected from the group consisting of breast cancer, prostate cancer, ovarian cancer, and benign prostate hyperplasia.
[E6] The combination according to any one of [E1] to [E5], wherein the tumor is a tumor resistant to endocrine therapy.
[E7] The combination according to any one of [E1] to [E6], wherein the tumor is breast cancer expressing FGFR1.
[E8] The combination according to any one of [E1] to [E7], wherein the tumor is breast cancer having FGFR1 gene amplification.
[E9] The combination according to any one of [E1] to [E8], wherein the tumor to which endocrine therapy is to be applied is breast cancer, and the endocrine therapy comprises at least one member selected from the group consisting of ovarian function suppression, aromatase inhibitor administration, antiestrogen administration, and progestogen administration.
[E10] The combination according to [E9], wherein the endocrine therapy is antiestrogen administration.
[E11] The combination according to [E10], wherein the antiestrogen is tamoxifen, fulvestrant, or a salt or derivative thereof.
[E12] The combination according to any one of [E1] to [E11], wherein endocrine therapy and administration of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof are performed simultaneously, separately, or continuously.
[E13] The combination according to any one of [E1] to [E12], wherein administration is performed on a tumor patient who has undergone endocrine therapy.
[E14] The combination according to any one of [E1] to [E13], wherein (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof is administered to a tumor patient who will undergo endocrine therapy after administration of the compound or a pharmaceutically acceptable salt thereof.

### Effects of Disclosure

According to the present disclosure, tumor treatment with high antitumor effects can be realized. Such effects include a tumor regression effect and a growth delay effect, which can lead to suppression of the progression of the symptoms of tumor patients, improvement of pathological conditions, and long-term survival.

### Brief Description of Drawings

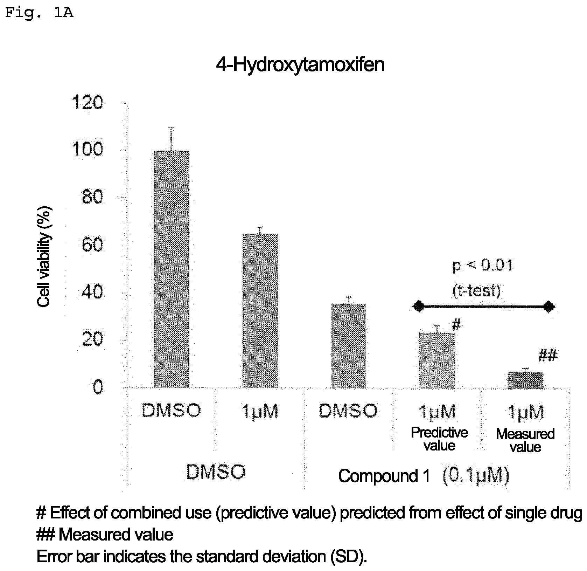

Fig. 1A shows the influence of the combined use of compound 1 and 4-hydroxytamoxifen in MDA-MB-134VI cells on cell growth.

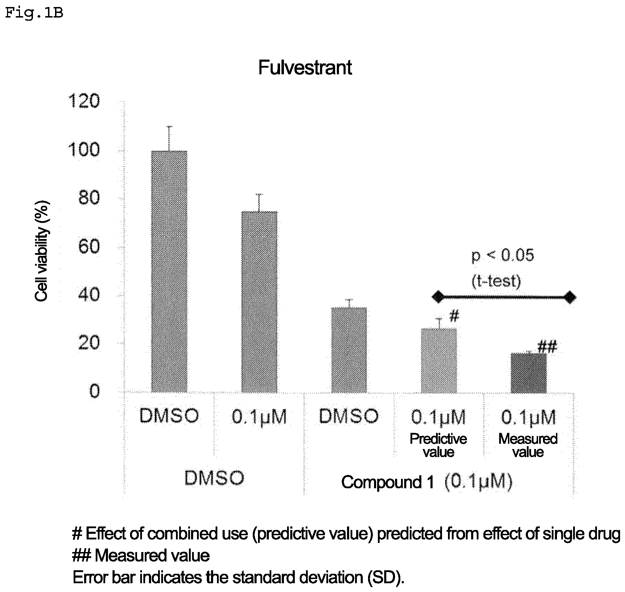

Fig. 1B shows the influence of the combined use of compound 1 and fulvestrant in MDA-MB-134VI cells on cell growth.

As embodiments, the present disclosure provides a pharmaceutical composition for use in treatment and/or prevention of tumors to which endocrine therapy is to be applied, the pharmaceutical composition comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof, and being used in combination with endocrine therapy, and also provides its related compound, use, method, and combination.

(S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one is a disubstituted benzene alkynyl compound having the following structure. In the present specification, this compound is simply referred to as "compound 1." Compound 1 is described as Example Compound 2 in PTL 1 (WO2013/108809) mentioned above. It has been reported that compound 1 has an excellent FGFR inhibitory effect and inhibits the ability of receptor protein tyrosine kinases (FGFR1, FGFR3, and FGFR4) to phosphorylate tyrosine in the substrate peptide sequence (PTL 1).

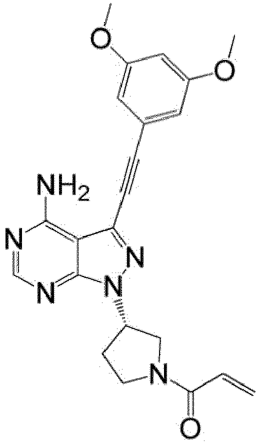

Compound 1 or a pharmaceutically acceptable salt thereof in the pharmaceutical composition of the present disclosure can be synthesized by any method; for example, it can be synthesized according to the production method described in WO2013/108809 (PTL 1).

In the present disclosure, compound 1 can be used as is or in the form of a pharmaceutically acceptable salt. The pharmaceutically acceptable salt of compound 1 is not particularly limited, and examples include addition salts with inorganic acids such as hydrochloric acid and sulfuric acid, or with organic acids such as acetic acid, citric acid, tartaric acid, and maleic acid; salts with alkali metals such as potassium and sodium; salts with alkaline earth metals such as calcium and magnesium; salts with organic bases such as ammonium salts, ethylamine salts, and arginine salts; and the like.

The present disclosure can be used for the treatment and/or prevention of tumors to which endocrine therapy is to be applied. The tumors to which endocrine therapy is to be applied include tumors expected to show some biological responses due to the application of endocrine therapy. Examples of some biological responses include changes in indicators (e.g., growth rate and stained image) at the cell or tissue level in the tumor samples, and changes in indicators (e.g., tumor volume reduction on images, overall survival (OS), progression-free survival (PFS), and response rate (RR)) in clinical trials. The tumors to which endocrine therapy is to be applied include tumors with some biological responses as a result of endocrine therapy. Further, the tumors to which endocrine therapy is to be applied include tumors resistant to endocrine therapy, in which patients have initially showed biological responses to endocrine therapy, but later the biological responses to endocrine therapy have diminished or disappeared.

The tumors to which endocrine therapy is to be applied include benign tumors, malignant tumors (cancer), and borderline malignant tumors.

The tumors to which endocrine therapy is to be applied include tumors in hormone target tissues. Hormone target tissues include the breast (lacteal gland), prostate, and ovary. Hormones include sex hormones. The tumors to which endocrine therapy is to be applied include hormone-dependent tumors. Sex hormones include sex steroid hormones.

The cancer to which endocrine therapy is to be applied includes breast cancer and prostate cancer. The cancer to which endocrine therapy is to be applied includes ovarian cancer.

An embodiment of the present disclosure is used for primary cancer and metastatic and recurrent cancer.

The benign tumors to which endocrine therapy is to be applied include benign prostate hyperplasia (BPH).

The treatment in the present disclosure includes procedures performed for the purpose of curing or ameliorating diseases, or for the purpose of suppressing disease progression or relapse or alleviating the symptoms. The treatment includes administration of drugs before or after surgical procedure. The treatment includes postoperative and preoperative therapies, as well as adjuvant and neoadjuvant therapies.

The prevention in the present disclosure includes suppression of the development of cancer to which endocrine therapy is to be applied without reaching the cancer to which endocrine therapy is to be applied. The prevention includes reducing the risk of developing cancer to which endocrine therapy is to be applied in patients who are at risk of developing the cancer to which endocrine therapy is to be applied.

The present disclosure is characterized in that compound 1 or a pharmaceutically acceptable salt thereof is used in combination with endocrine therapy. "Endocrine therapy" is also called "hormone therapy," and is a prevention and treatment method that works to suppress the development of tumors by directly or indirectly controlling hormonal stimulation of the tumors.

Some types of breast cancer are known to be progressed by the presence of estrogen, and these types of breast cancer have a corresponding expression of estrogen receptor and/or progesterone receptor (ER- and/or PgR-positive). Endocrine therapy in breast cancer is a treatment or prevention method that suppresses the progression of malignant tumors due to the presence of estrogen.

Main types of endocrine therapy in breast cancer can be roughly divided into ovarian function suppression (OFS), administration of aromatase inhibitors, administration of antiestrogens, and administration of progestogens.

Examples of ovarian function suppression include surgical procedures including oophorectomy, radiation procedures including elimination of ovarian function by irradiation, and administration of LH-RH agonists. LH-RH agonists suppress the action of pituitary hormones that promote estrogen biosynthesis in the ovaries, and reduce the ability of the ovaries to produce estrogen. LH-RH agonists include, for example, goserelin, leuprorelin, triptorelin, and salts and derivatives thereof.

Aromatase inhibitors reduce the ability to produce estrogen by inhibiting aromatase, which produces estrogen from male hormones. Aromatase inhibitors include, for example, anastrozole, exemestane, letrozole, and salts and derivatives thereof.

Antiestrogens include, for example, selective estrogen receptor modulators that inhibit the binding of estrogen to the estrogen receptor, and selective estrogen receptor down-regulators that have binding inhibitory activity and estrogen receptor degradation-promoting activity. Examples of the former include tamoxifen, toremifene, raloxifene, and salts and derivatives thereof. Examples of the latter includes fulvestrant, and salts and derivatives thereof.

Progestogens exert antitumor effects on breast cancer through various actions including anti-estrogen action. Progestogens include, for example, medroxyprogesterone acetate (a synthetic progestogen), and salts and derivatives thereof.

An embodiment of the present disclosure is used for premenopausal breast cancer. Further, an embodiment of the present disclosure is used for postmenopausal breast cancer.

An embodiment of the present disclosure is used for estrogen receptor- and/or progesterone receptor-positive (ER- and/or PgR-positive) breast cancer. ER and/or PgR positivity can be determined, for example, by a determination method by the occupancy of positive cells using an immunohistochemical method (IHC method), or a method combining occupancy and staining intensity. In the case of determination by occupancy, the presence of at least one positive cell (any positive cell) can be used as an index. In addition, the occupancy of positive cells of 1% or 10% can be used as a cutoff index. Further, staining intensity can also be taken into consideration (Arch Pathol Lab Med. 2010; 134 (7): e48-72). ER and/or PgR positivity can also be determined by methods that can show results scientifically similar to those of the methods mentioned above. Examples of ER- and/or PgR-positive breast cancer include not only breast cancer that tests positive at the primary stage, but also breast cancer that tests positive for ER and/or PgR at least once at any stage in the evolution of breast cancer, including metastasis and recurrence. Receptors for estrogen include two highly homologous isoforms, ERα and ERβ, which are encoded by different genes, ESR1 and ESR2, respectively (Arch Pathol Lab Med., ibid). When referred to simply as estrogen receptor or ER in the present specification, it means ERα (ESR1 gene product).

An embodiment of the present disclosure is used for prostate cancer. Some types of prostate cancer are known to be progressed by the presence of androgen, and these types of prostate cancer have a corresponding androgen receptor expression (AR-positive). Endocrine therapy in prostate cancer is a treatment or prevention method that suppresses the progression of malignant tumors due to the presence of androgen.

The main types of endocrine therapy in prostate cancer can be roughly divided into suppression of secretion of testosterone, which is a type of androgen (resection of the testes, which are reproductive organs, or drug administration of LH-RH agonists, LH-RH antagonists, estrogenic drugs, or the like), and endocrine therapy such as administration of antiandrogens, androgen synthesis inhibitors, etc., for androgen-dependent prostate cancer.

LH-RH agonists act on the pituitary gland to reduce testosterone production. LH-RH agonists include, for example, goserelin, leuprorelin, buserelin, and salts and derivatives thereof.

Some of the known mechanisms of antiandrogens are, for example, inhibition of ligand binding to the androgen receptor, inhibition of nuclear translocation of the androgen receptor, and inhibition of androgen receptor signaling. Antiandrogens include, for example, chlormadinone acetate, bicalutamide, flutamide, enzalutamide, nilutamide, the compounds disclosed in WO2015/182712, and salts and derivatives thereof.

Further, endocrine therapy includes, for example, androgen synthesis inhibitors that inhibit enzymes on the androgen synthesis pathway. Such androgen synthesis inhibitors include, for example, ketoconazole, aminoglutetide, abiraterone acetate, and salts and derivatives thereof.

In an embodiment of the present disclosure, compound 1 or a pharmaceutically acceptable salt thereof is administered for the treatment and/or prevention of tumors to which endocrine therapy is to be applied, in combination with endocrine therapy that is administration of the compound disclosed in WO2015/182712, which is an antiandrogen. In an embodiment of the present disclosure, compound 1 or a pharmaceutically acceptable salt thereof is administered for the treatment and/or prevention of tumors to which endocrine therapy is to be applied, in combination with endocrine therapy that is administration of an antiandrogen other than the compound disclosed in WO2015/182712.

Surgical procedures, radiation procedures, and drug administration in the endocrine therapy described above are known, and these procedures can be performed by persons skilled in the art. The mechanism of action, dose, and treatment target of the above drugs are known and can be obtained together with necessary information on the drugs. When the endocrine therapy is drug administration, persons skilled in the art can administer an effective amount of the drug.

An embodiment of the present disclosure is used for ovarian cancer. In an embodiment, compound 1 or a pharmaceutically acceptable salt thereof is used in combination with an LH-RH agonist for ovarian cancer.

An embodiment of the present disclosure is used for benign prostate hyperplasia (BPH). In an embodiment, compound 1 or a pharmaceutically acceptable salt thereof is used in combination with an antiandrogen for benign prostate hyperplasia (BPH).

An embodiment of the present disclosure is used for tumors expressing FGFR. In an embodiment, the FGFR is selected from the group consisting of FGFR1, FGFR2, FGFR3, and FGFR4. In a preferable embodiment, the FGFR is FGFR1.

The expression of FGFR can be detected by methods known to persons skilled in the art. Gene expression and gene product proteins can be detected by known methods, such as methods using antibodies (immunohistochemistry, enzyme immunoassay (ELISA), flow cytometry, immunoblotting, etc.), methods using nucleic acids (in situ hybridization, PCR, Northern blotting, etc.), and methods based on a common principle of these methods. The detection device can be any known device (GeneChip, microarray, etc.).

Further, an embodiment of the present disclosure is used for tumors with activated FGFR signaling. FGFRs bind to FGFs to form dimers and are activated by phosphorylation, which induces recruitment and activation of specific downstream signal transduction molecules, thereby developing physiological functions.

Activation of FGFR signaling can be detected by methods known to persons skilled in the art. Signaling activation can be detected, for example, by detecting FGFs, which are substrates for FGFRs; by detecting direct phosphorylation state of FGFRs, which are phosphorylated enzymes, or biological activity including enzymatic activity; or by detecting phosphorylation state of intracellular substrates and intracellular proteins existing in the FGFR signaling cascade downstream, or biological activity including enzymatic activity.

Further, an embodiment of the present disclosure is used for tumors having FGFR genetic aberrations. FGFR genetic aberrations include gene amplification, gene mutation, chromosomal translocation, insertion, and inversion, and gene fusion. Some of the FGFR genetic aberrations in tumors have already been reported in documents available to persons skilled in the art (NPL 11 and NPL 12). FGFR1 genetic aberrations can be detected by methods known to persons skilled in the art, for example, pyrosequence, DNA sequencing including next-generation sequencing (NGS), PCR-based methods including allele-specific PCR chain reactions, microarray-based comparative genomic hybridization (aCGH), fluorescence *in situ* hybridization (FISH), and chromogenic *in situ* hybridization (CISH).

An embodiment of the present disclosure is used for breast cancer with FGFR1 gene amplification. The breast cancer with FGFR1 gene amplification refers to breast cancer in which the copy number of FGFR1 gene in the genome is higher than that of normal cells. FGFR1 gene amplification can be detected by methods known to persons skilled in the art, for example, DNA sequencing including next generation sequencing (NGS), microarray-based comparative genomic hybridization (aCGH), fluorescence *in situ* hybridization (FISH), and chromogenic in situ hybridization (CISH).

In the present disclosure, two procedures, i.e., administration of compound 1 or a pharmaceutically acceptable salt thereof and endocrine therapy, are used in combination. The phrase "used in combination" means that these two procedures are performed simultaneously, separately, or continuously in a series of treatment processes. Therefore, the present disclosure includes an embodiment in which compound 1 or a pharmaceutically acceptable salt thereof is administered to tumor patients who have already undergone endocrine therapy. Further, the present disclosure includes an embodiment in which compound 1 or a pharmaceutically acceptable salt thereof is administered simultaneously with endocrine therapy. In this case, when the endocrine therapy is performed by drug administration, the drug of the endocrine therapy and compound 1 or a pharmaceutically acceptable salt thereof can be formed into a single pharmaceutical preparation or a single administration kit. Moreover, the present disclosure includes an embodiment in which, for example, endocrine therapy is performed on tumor patients who have already been administered with compound 1 or a pharmaceutically acceptable salt thereof. When the endocrine therapy is performed by drug administration, the administration interval when the drug of the endocrine therapy and compound 1 or a pharmaceutically acceptable salt thereof are separately administered is not particularly limited, and can be selected so that the effect of each of compound 1 or a pharmaceutically acceptable salt thereof and the other compound having an antitumor effector a pharmaceutically acceptable salt thereof, and the effect of combined use can be optimally exhibited.

The present disclosure includes an embodiment in which administration of other antitumor agents and other procedures are performed, in addition to the combined use of the two procedures, i.e., administration of compound 1 or a pharmaceutically acceptable salt thereof and endocrine therapy. In the present disclosure, the endocrine therapy used in combination with the administration of compound 1 or a pharmaceutically acceptable salt thereof may be performed a single time, or the same or different types of endocrine therapy may performed multiple times.

In an embodiment of the present disclosure, compound 1 or a pharmaceutically acceptable salt thereof enhances the antitumor effect of the endocrine therapy used in combination. An embodiment of the present disclosure provides a pharmaceutical composition comprising compound 1 or a pharmaceutically acceptable salt thereof for use in enhancing the antitumor effect of endocrine therapy. An embodiment of the present disclosure provides compound 1 or a pharmaceutically acceptable salt thereof for use in enhancing the antitumor effect of endocrine therapy. An embodiment of the present disclosure provides use of compound 1 or a pharmaceutically acceptable salt thereof for the manufacture of a medicament for use in enhancing the antitumor effect of endocrine therapy. An embodiment of the present disclosure provides a method for enhancing the antitumor effect of endocrine therapy, comprising administering an effective amount of compound 1 or a pharmaceutically acceptable salt thereof to a patient. An embodiment of the present disclosure provides a combination of endocrine therapy and administration of compound 1 or a pharmaceutically acceptable salt thereof for enhancing the antitumor effect of endocrine therapy.

In the present disclosure, compound 1 or a pharmaceutically acceptable salt thereof and the drug used in the endocrine therapy may be formed into multiple separate preparations, or may be integrated into a single preparation. In addition, the present disclosure may further contain active ingredients other than compound 1 or pharmaceutically acceptable salts thereof and the other compound having an antitumor effect or pharmaceutically acceptable salts thereof.

When compound 1 or a pharmaceutically acceptable salt thereof and the other drug used in endocrine therapy are incorporated in a pharmaceutical preparation as active ingredients, each active ingredient can be optionally mixed with a pharmaceutical carrier, thereby forming various dosage forms according to prevention and treatment purposes. Examples of the dosage form include oral preparations, injections, suppositories, ointments, patches, and the like. Of these, oral preparations are preferable. Examples of oral preparations include tablets, capsules, granules, powders, syrups, and the like, without any limitation. Such dosage forms can be formed by methods conventionally known to persons skilled in the art. A suitable carrier, such as an excipient, diluent, bulking agent, or disintegrant, can be optionally added to the pharmaceutical preparation or pharmaceutical composition according to the dosage form.

Persons skilled in the art can administer an effective amount of compound 1 or a pharmaceutically acceptable salt thereof to a patient. The amount of compound 1 or a pharmaceutically acceptable salt thereof to be incorporated in each of such dosage unit forms depends on the condition of the patient to whom compound 1 or its salt is administered, the dosage form, etc. In general, in the case of an oral preparation, an injection, and a suppository, the amount is preferably 0.05 to 1000 mg, 0.01 to 500 mg, and 1 to 1000 mg, respectively, per dosage unit form.

The dose of compound 1 or a pharmaceutically acceptable salt thereof per day depends on the condition, body weight, age, gender, etc. of the patient, and cannot be generalized. For example, the dose of compound 1 or a pharmaceutically acceptable salt thereof for an adult (body weight: 60 kg) per day is typically about 1 to 1000 mg, preferably about 10 to 500 mg, and more preferably about 20 to 300 mg.

When compound 1 or a pharmaceutically acceptable salt thereof is administered every day, the dose of compound 1 or a pharmaceutically acceptable salt thereof is, for example, about 1 to 200 mg, preferably 2 to 100 mg, more preferably 4 to 50 mg, and even more preferably 10 to 40 mg, per day.

When compound 1 or a pharmaceutically acceptable salt thereof is administered on alternate days, the dose of compound 1 or a pharmaceutically acceptable salt thereof is, for example, about 2 to 1000 mg, preferably 10 to 500 mg, more preferably 20 to 200 mg, and even more preferably 50 to 160 mg, per day.

In the present disclosure, the preparation comprising compound 1 or a pharmaceutically acceptable salt thereof and the drug used in the endocrine therapy may be administered via the same or different routes. For example, the preparation comprising compound 1 or a pharmaceutically acceptable salt thereof and the drug used in the endocrine therapy can both be orally administered. Alternatively, for example, the preparation comprising compound 1 or a pharmaceutically acceptable salt thereof can be orally administered, and the drug used in the endocrine therapy can be injected intravenously. The administration route can be suitably determined depending on the active ingredient to be administered, and in consideration of the stage of malignant tumor progression in the patient, the overall status of the patient, and the like.

In the present disclosure, compound 1 or a pharmaceutically acceptable salt thereof can be administered to patients before or after surgery, and can also be administered to inoperable patients. Further, the present disclosure may include administration of drugs for further enhancing the antitumor effect, and administration of drugs for reducing the side effects.

As an embodiment of the present disclosure, for example, for the treatment of breast cancer, compound 1 or a pharmaceutically acceptable salt thereof can be used in combination with an LH-RH agonist, an aromatase inhibitor, an antiestrogen and/or a progestogen. Both drugs can be orally administered as active ingredients, or as active ingredients of the pharmaceutical composition described in the present disclosure.

As an embodiment of the present disclosure, for example, for the treatment of prostate cancer, compound 1 or a pharmaceutically acceptable salt thereof can be used in combination with an LH-RH agonist, an LH-RH antagonist, an estrogenic drug, an antiandrogen and/or an androgen synthesis inhibitor. Both drugs can be orally administered as active ingredients, or as active ingredients of the pharmaceutical composition described in the present disclosure.

The administration or mixing ratio of compound 1 or a pharmaceutically acceptable salt thereof and the drug used in the endocrine therapy is not particularly limited insofar as antitumor effects are exerted. For example, the amount of compound 1 or a pharmaceutically acceptable salt thereof as a free form may be about 0.001 to 1000 moles, and preferably about 0.01 to 100 moles, per mole of the other compound having an antitumor effect.

For example, when the other compound having an antitumor effect is an antiestrogen, the suitable range of compound 1 or a pharmaceutically acceptable salt thereof used is 0.001 to 10 moles, preferably 0.001 to 1 mole, and more preferably 0.001 to 0.1 moles, per mole of the antiestrogen.

The present disclosure can also be effectively used for tumors resistant to FGFR inhibitors other than compound 1 or pharmaceutically acceptable salts thereof.

### Examples

The present disclosure is described below in more detail with reference to Examples. However, the scope of the present disclosure is not limited to these Examples. The present disclosure is fully described below by way of Examples; however, it is understood that various changes and modifications by a skilled artisan are possible. Therefore, such changes and modifications are included in the present disclosure as long as they do not depart from the scope of the disclosure. The various reagents used in the Examples were obtained from commercial suppliers, unless otherwise specified.

### Example 1: Evaluation of Cell Growth-Inhibitory Effect by Combined Use of Compound 1 and 4-Hydroxytamoxifen or Fulvestrant In Vitro (Combination Index)

### A. Material and Method

Human breast cancer cell line T47D (Dainippon Pharmaceutical Co., Ltd.) was cultured in RPMI-1640 medium containing 10% fetal bovine serum with reference to British Journal of Cancer (2006) 94, 661-671. The cells were maintained at 37°C and 5% CO₂, and passaged once or twice per week at a ratio of 1:5 to 1:20. Human breast cancer cell line MDA-MB-134VI (American Type Culture Collection) was cultured in RPMI-1640 medium containing 10% fetal bovine serum with reference to British Journal of Cancer (2006) 94, 661-671. The cells were maintained at 37°C and 5% CO₂, and passaged once or twice per week at a ratio of 1:2 to 1:3.

### Cell Viability Assay

The cell viability was measured using CellTiter-Glo 2.0 (Promega). After being collected according to a usual method, the cells were suspended in RPMI-1640 (phenol red-free) medium containing 1% fetal bovine serum (Charcoal Stripped), 0.5 ng/mL estradiol, and 25 ng/mL human FGF2, and seeded on a 96-well ultralow attachment plate. The number of cells seeded was 500 cells/80 µL per well. After incubation at 37°C and 5% CO₂ for 24 hours, 10 µL of medium containing a drug solution was added. Drug solutions were prepared for a vehicle (DMSO) group and a dilution series of single drugs (compound 1, tamoxifen, and fulvestrant). Experiments were carried out on 7 groups in total: the vehicle group, single drug groups of compound 1, tamoxifen, and fulvestrant, and their combination groups (compound 1 + vehicle, compound 1 + tamoxifen, and compound 1 + fulvestrant). The maximum final concentration of compound 1 was 1 µM, and 5 concentrations were set for a 10-fold dilution series. For 4-hydroxytamoxifen and fulvestrant, a 10-fold dilution series with a maximum concentration of 10 µM was set for 4 concentrations. After drug addition, the cells were further incubated at 37°C and 5% CO₂ for 120 hours. Experiments were carried out in duplicate (2 wells per treatment group). The cell viability was calculated by adding 100 µL of CellTiter-Glo 2.0 reagent per well, incubating at room temperature for 30 minutes, and then measuring the chemiluminescence of each well with a plate reader (EnSpire). The cell viability in the case of drug addition was calculated as a ratio with respect to the control group, which was set to 100%, according to the following formula, and the average value of the data of two wells was used for the analysis.

### Cell viability (%) = (chemiluminescence in the case of drug addition)/(chemiluminescence of control group) × 100

The Fa (fraction of affect) value was calculated by subtracting 1/100 of the cell viability from 1.

Next, the combination index (CI) value was determined at combined concentrations of the drugs. The effect of combined use of two drugs was determined as follows: a CI value of more than 1, equal to 1, or less than 1 indicated antagonism, addition, or synergism, respectively (Table 1, Pharmacol Rev. 2006; 58 (3): 621-81, BMC Complement Altern Med. 2013; 13: 212, Anticancer Res. 2005; 25 (3B): 1909-17).

**Table 1**

| Range of CI (upper limit) | Explanation |
|---|---|
| 0.1 | Very strong synergism |
| 0.3 | Strong synergism |
| 0.7 | Synergism |
| 0.85 | Moderate synergism |
| 0.9 | Slight synergism |
| 1 | Almost additive |
| 1.2 | Slight antagonism |
| 1.45 | Moderate antagonism |
| 3.3 | Antagonism |
| 10 | Strong antagonism |
| >10 | Very strong antagonism |

Further, from the Fa values calculated by the concentration combinations of compound 1 and 4-hydroxytamoxifen or fulvestrant in the T47D cells and MDA-MB-134VI cells, concentration combinations of both drugs satisfying Fa = 0.5 (corresponding to ED50), 0.75 (corresponding to ED75), and 0.9 (corresponding to ED90) were extracted and subjected to linear curve fitting by CalcuSyn (HULINKS Inc.) to obtain CI.

### B. Results

In the T47D breast cancer cell line expressing FGFR1, and the MDA-MB-134VI breast cancer cell line having FGFR1 gene amplification, the combined use of compound 1 and 4-hydroxytamoxifen showed synergism in all of the combinations of the four concentration ratios performed (Table 2). The following Table 2 shows the combination index values when the ratio of 4-hydroxytamoxifen and compound 1 was 1:1, 10:1, 100:1, and 1000:1 at each time point in which the cell growth-inhibitory effect in combined use was 50%, 75%, and 90% (ED50, ED75, and ED90).

**Table 2**

| **Cell line** | **4-Hydroxytamoxifen** : **compound 1** | ED50 | ED75 | ED90 |
|---|---|---|---|---|
| T47 D | 1:1 | 0.27 | 1.90 | 14.49 |
| | 10:1 | 0.19 | 0.31 | 0.53 |
| | 100:1 | 0.38 | 0.45 | 0.54 |
| | 1000:1 | 0.40 | 0.51 | 0.65 |
| MDA-MB-134VI | 1:1 | 0.40 0.14 | 0.13 | 0.65 0.28 |
| | 10:1 | 0.32 | 0.26 | 0.37 |
| | 100:1 | 0.52 | 0.61 | 0.78 |
| | 1000:1 | 0.66 | 0.72 | 0.79 |
| | | | | |

| **Cell line** | **Fulvestrant: compound 1** | ED50 | ED75 | ED90 |
|---|---|---|---|---|
| T47D | 1:1 | 0.01 | 0.01 | 0.01 |
| | 10:1 | 0.03 | 0.03 | 0.03 |
| | 100:1 | 0.05 | 0.02 | 0.01 |
| | 1000:1 | 0.16 | 0.08 | 0.04 |
| MDA-MB-134VI | 1:1 | 0.11 | 0.04 | 0.02 |
| | 10:1 | 0.18 | 0.04 | 0.01 |
| | 100:1 | 0.13 | 0.01 | 0.00 |
| | 1000:1 | 0.46 | 0.09 | 0.02 |

### Example 2: Evaluation of Cell Growth-Inhibitory Effect by Combined Use of Compound 1 and 4-Hydroxytamoxifen or Fulvestrant

### In Vitro

### A. Material and Method

Human breast cancer cell line MDA-MB-134VI (American Type Culture Collection) was cultured in RPMI-1640 medium containing 10% fetal bovine serum with reference to British Journal of Cancer (2006) 94, 661-671. The cells were maintained at 37°C and 5% CO₂, and passaged once or twice per week at a ratio of 1:2 to 1:3.

### Cell Viability Assay

The cell viability was measured using CellTiter-Glo 2.0 (Promega). After being collected according to a usual method, the cells were suspended in RPMI-1640 (phenol red-free) medium containing 1% fetal bovine serum (Charcoal Stripped), 0.5 ng/mL estradiol, and 25 ng/mL human FGF2, and seeded on a 96-well ultralow attachment plate. The number of cells seeded was 500 cells/80 µL per well. After incubation at 37°C and 5% CO₂ for 24 hours, 10 µL of medium containing compound 1 and 4-hydroxytamoxifen or fulvestrant, and as a control, 10 µL of medium containing a vehicle (DMSO) containing none of these drugs were added. The maximum concentration was set to 0.1 µM for compound 1, 1 µM for 4-hydroxytamoxifen, and 0.1 µM for fulvestrant. After drug addition, the cells were further incubated at 37°C and 5% CO₂ for 120 hours. Experiments were carried out in triplicate (3 wells per treatment group). The cell viability was calculated by adding 100 µL of CellTiter-Glo 2.0 reagent per well, incubating at room temperature for 30 minutes, and then measuring the chemiluminescence of each well with a plate reader (EnSpire). The cell viability in the case of drug addition was calculated as a ratio with respect to the control group, which was set to 100%, according to the following formula, and the average value of the data of three wells was used for the analysis.

### Cell viability (%) = (chemiluminescence in the case of drug addition)/(chemiluminescence of control group) × 100

### B. Results

The treatment with compound 1 (0.1 µM), 4-hydroxytamoxifen (1 µM), or fulvestrant (0.1 µM) alone suppressed the growth of the MDA-MB-134VI cells, and T/C ((treated (treatment group)/control (control group)) (%) on the day of determination was 35%, 65%, and 75%, respectively. In contrast, the combined treatment with a combination of 0.1 µM of compound 1 and 1 µM of 4-hydroxytamoxifen, and the combined treatment with a combination of 0.1 µM of compound 1 and 0.1 µM of fulvestrant further suppressed the cell growth, compared with the treatment with each drug alone, and T/C (%) was 7% and 16%, respectively.

The cell growth-inhibitory effect of the combined use of compound 1 and 4-hydroxytamoxifen or fulvestrant exceeded effects of the combined use of both drugs (T/C = 23% and 27%) computationally predicted based on the effect of treatment with each drug alone as an index (Bliss method, Ann Appl Biol. 1939; 26: 585-615). This clarified that synergistic effects of the combined use were exhibited (P<0.05; Student's t-test) (Figs. 1A and 1B) .

## Claims

1. A pharmaceutical composition for use in treatment and/or prevention of a tumor to which endocrine therapy is to be applied, the pharmaceutical composition comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof, and the pharmaceutical composition being administered in combination with endocrine therapy.

2. The pharmaceutical composition according to claim 1, wherein the tumor is a tumor expressing FGFR.

3. The pharmaceutical composition according to claim 1 or 2, wherein the tumor is a tumor with activated FGFR signaling.

4. The pharmaceutical composition according to any one of claims 1 to 3, wherein the tumor is a tumor having an FGFR genetic aberration.

5. The pharmaceutical composition according to any one of claims 1 to 4, wherein the tumor to which endocrine therapy is to be applied is selected from the group consisting of breast cancer, prostate cancer, ovarian cancer, and benign prostate hyperplasia.

6. The pharmaceutical composition according to any one of claims 1 to 5, wherein the tumor is a tumor resistant to endocrine therapy.

7. The pharmaceutical composition according to any one of claims 1 to 6, wherein the tumor is breast cancer expressing FGFR1.

8. The pharmaceutical composition according to any one of claims 1 to 7, wherein the tumor is breast cancer having FGFR1 gene amplification.

9. The pharmaceutical composition according to any one of claims 1 to 8, wherein the tumor to which endocrine therapy is to be applied is breast cancer, and the endocrine therapy comprises at least one member selected from the group consisting of ovarian function suppression, aromatase inhibitor administration, antiestrogen administration, and progestogen administration.

10. The pharmaceutical composition according to claim 9, wherein the endocrine therapy is antiestrogen administration.

11. The pharmaceutical composition according to claim 10, wherein the antiestrogen is tamoxifen, fulvestrant, or a salt or derivative thereof.

12. The pharmaceutical composition according to any one of claims 1 to 11, wherein endocrine therapy and administration of the composition comprising (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof are performed simultaneously, separately, or continuously.

13. The pharmaceutical composition according to any one of claims 1 to 12, which is administered to a tumor patient who has undergone endocrine therapy.

14. The pharmaceutical composition according to any one of claims 1 to 13, which is administered to a tumor patient who will undergo endocrine therapy after administration of the pharmaceutical composition.

15. (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof for use in treatment and/or prevention of a tumor to which endocrine therapy is to be applied, being administered in combination with endocrine therapy.

16. Use of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof for the manufacture of a medicament for use in treatment and/or prevention of a tumor to which endocrine therapy is to be applied, being administered in combination with endocrine therapy.

17. A method for treating and/or preventing a tumor to which endocrine therapy is to be applied, comprising administering an effective amount of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof to a patient, in combination with endocrine therapy.

18. A combination of endocrine therapy and administration of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a pharmaceutically acceptable salt thereof, for use in treatment and/or prevention of a tumor to which endocrine therapy is to be applied.
